# EUROPEAN PATENT SPECIFICATION

(11) **EP 2 083 806 B1**
(45) Date of publication and mention of the grant of the patent: **11.01.2012**
(21) Application number: 07834529.5
(22) Date of filing: 20.11.2007
(51) Int. Cl.: A61K 31/122, A61K 36/9066, C07C 45/83, A61P 35/00

(54) **USE OF AN ANTI-CANCER COMPOUND**
VERWENDUNG EINER ANTIKREBSVERBINDUNG
UTILISATION D'UN COMPOSÉ ANTI-CANCER

(30) Priority: 21.11.2006 MY 0604536
(43) Date of publication of application: 05.08.2009
(73) Proprietor: Universiti Putra Malaysia, 43400 Upm Serdang, Selangor (MY)
(72) Inventor: ABDUL, Ahmad Bustamam, 43400 UPM Serdang Selangor (MY); ABDULLAH, Munammad Nazrul Hakim, 43400 UPM Serdang, Selangor (MY); LAJIS, Mohd Nordin, 43400 UPM Serdang Selangor (MY); TAILAN, Nirmala Devi, 43400 UPM Serdang, Selangor (MY); MOHD ZAIN, Zetty Nadia, 43400 UPM Serdang Selangor (MY); WAHAB, Siddig Ibrahim Abdel, 43400 Serdang (MY); AL-ZUBAIRI, Adel, Sharaf, 43400 Serdang (MY)
(74) Representative: Bates, Philip Ian
(86) International application number: PCT/MY2007/000082
(87) International publication number: WO 2008/063045

(56) References cited:
- WO-A-92/15543
- JP-A- 11 049 793
- US-A1- 2006 083 799
- TAKADA Y. ET AL.: 'Zerumbone abolishes NF-kappaB and IkappaBa kinase activation leading to suppression of antiapoptotic and metastatic gene expression, upregulation of apoptosis, and downregulation of invasian' ONCOGENE vol. 24, no. 46, 2005, pages 6957 - 6969, XP008100998
- MURAKAMI A. ET AL.: 'Zerumbone, a sesquiterpene in subtropical ginger, suppresses skin tumor initiation and promotion stages in ICR mice' INTERNATIONAL JOURNAL OF CANCER vol. 110, no. 4, 2004, pages 481 - 490, XP008101000
- KIRANA C. ET AL.: 'Antitumor activity of extract of Zingiber aromaticum and its bioactive sesquiterpenoid Zerumbone' NUTRITION AND CANCER vol. 45, no. 2, 2003, pages 218 - 225, XP008101183
- HOFFMANN A. ET AL.: 'Letter to the editor: redox-regulated mechanism may account for Zerumbone's ability to suppress cancer-cell proliferation' CARCINOGENESIS vol. 23, no. 11, 2002, page 1961, XP008101001
- MURAKAMI A. ET AL.: 'Zerumbone, a southeast Asian giner sesquiterpene, markedly suppresses free radical generation, proinflammatory protein production, and cancer cell proliferation accompanied by apoptosis: The alpha,beta-unsaturated carbonyl group is a prerequisite' CARCINOGENESIS vol. 23, no. 5, 2002, pages 795 - 802, XP008101002

## Description

The present invention relates to a therapeutic natural compound and the medical use thereof. More particularly, the present invention relates to a natural compound known as zerumbone as an active agent for the manufacture of a pharmaceutical preparation for treating cervical and ovarian carcinoma. The present invention also relates to a method of extracting the compound and a pharmaceutical composition containing the compound.

### BACKGROUND TO THE INVENTION

Cancer refers collectively to a family of diseases that start mainly from the uncontrolled proliferation of cells, invades neighboring normal tissues or organs and establishes a new growth place therein that can eventually cause death. In recent years, conspicuous advances have been made in the regulation of cell cycle or apoptosis and in the development of novel targets including oncogenes and tumor suppressor genes. However, despite these efforts, the incidence of cancer keeps increasing.

The three most common types of cancer treatment are surgery, radiotherapy and chemotherapy. Treatment is aimed at removing the cancer cells or destroying them in the body with medicines or other agents.

Surgery can be very successful in treating some kinds of cancer, but it is not an option for all people. If the cancer is in the form of a malignant tumor and the tumor is in one place (localized), it may be possible to safely "cut out" the tumor and any surrounding affected tissue. Surgery may not be possible if the cancer has spread to other areas of the body or if the tumor cannot be removed without damaging vital organs, such as the liver or brain.

Radiotherapy uses radiation - in the form of a special kind of x-ray, gamma rays or electrons - to damage cancer cells so that they can't multiply. There is usually no pain during therapy. Radiotherapy may sometimes be the only treatment needed, or it may be used with other therapies, such as surgery. A combination of surgery and radiotherapy may be used for tumors that grow in one place.

Chemotherapy uses medicines to attack the cancer cells. Just the word "chemotherapy" can cause a lot of fear because the side effects can be severe. However, not all people experience severe side effects. The side effects of chemotherapy can often be reduced with other medicines. Chemotherapy is usually used when the cancer has spread to other areas in the body. Chemotherapy can also be used in combination with surgery and radiation. Sometimes the tumor is surgically removed and then chemotherapy is used to make sure all the cancer cells are killed. One drawback of chemotherapy is that it is often not effective in the treatment of general epithelial-derived tumors, such as breast, colon and lung cancer. This is mainly due to the resistance of cancer cells to several anti-caner agents, leading to very limited application of the anti-cancer agents.

Most currently available anti-cancer agents were developed based on the fact that cancer cells divide more rapidly than normal cells, and were exemplified by compounds inhibiting DNA replication or synthesis, metabolism inhibitors, cell division inhibitors, nucleotide analogues and topoisomerase inhibitors. Thus, anti-cancer agents typically effect the division and survival of cancer cells. Since conventional anti-cancer agents are focused on the rapid division of cancer cells, they have disadvantages that include having toxicity (e.g. body hair fall out) and effecting normal cells that rapidly divide under normal conditions (e.g. bone marrow cells).

Another kind of treatment is biological therapy. This treatment uses proteins to trigger the body's immune system to produce more white blood cells (or lymphocytes). Two lymphocytes that can attack and kill cancer cells are the T-cell and the B-cell. The proteins boost the ability of the T-cell and B-cell lymphocytes to kill cancer. Biological therapy can also be used in combination with surgery, radiation therapy or chemotherapy.

Hormone therapy is sometimes used to treat breast or prostate cancer. The hormone estrogen can make breast cancer tumors grow faster. Similarly, the hormone testosterone can make cancerous tumors in the prostate grow faster. Drugs that contain other hormones may be used to block the effects of estrogen and testosterone. In other cases, surgery to remove the ovaries or the testicles may be used. Removing these organs reduces the amount of estrogen or testosterone in the body. Hormone therapy is often used in addition to chemotherapy or radiotherapy. Botanical extracts, are believed to have therapeutic potential in the treatment and prevention of various diseases. The rhizomes of *Zingiber zerumbet* are a well-known crude drug used for anti-inflammatory folk medicine (Elliot et al., 1987). *Z*. *zerumbet* belongs to the Zingiberaceae (ginger family) with common names of pinecone ginger, shampoo ginger, Awapuhi kuahiwi (Hawaii) and 'lempoyang' (Malaysia). In general, several species of Zingiberaceae are used as spices, medicines, flavouring agents and as a source of certain dyes in the Southeast Asian region (Burkill, 1966).

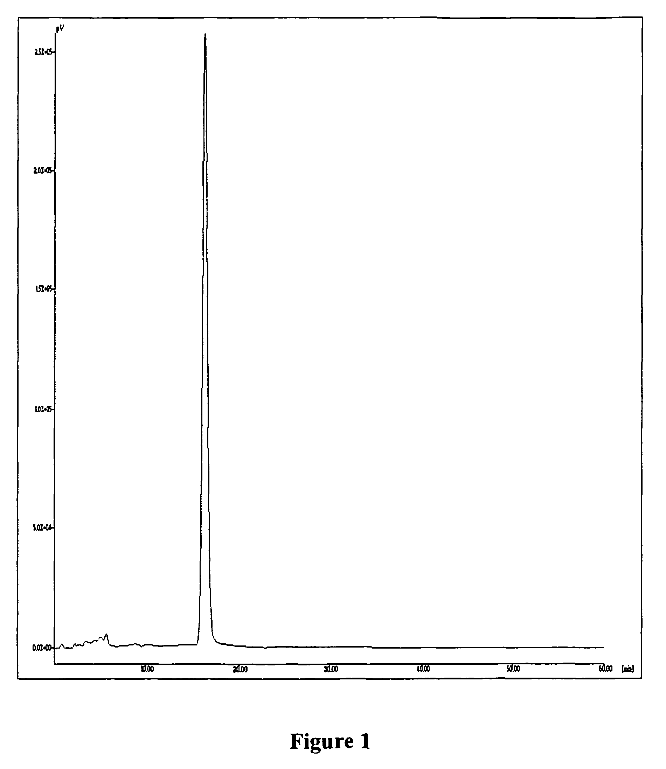

Zerumbone, the main component of the essential oil of a wild ginger, *Zingiber zerumbet* is a monocyclic sesquiterpene containing a cross-conjugated dienone system (Kitayama et al., 2002) (Figure 1). A large amount of the archiral sesquiterpene zerumbone was detected in rhizomes, which are used locally as anti-inflammatory medicines (D'Odorico et al., 2001). This compound is also present in some edible parts of the plant, in particular, the young stems and inflorescence, which are used in traditional cooking (Kankuri et al., 1999).

Matthes et al. (1980) reported that zerumbone is cytotoxic whilst Murakami et al. (1999) stated that zerumbone is a potent inhibitor of 12-O-tetradeconyl 13-acetate-induced Epstein-Barr virus activation. Interestingly, zerumbone was found to inhibit inducible nitric oxide synthase (iNOS) and COX-2 expression in RAW264.7 macrophages treated with lipopolysaccharide (LPS) and interferon (IFN)-□, and nitric oxide (NO)/02- generation in leukocytes (Tanaka et al., 2001). Zerumbone was also shown to suppress TNF-□ release and also induces apoptosis in a variety of human colonic adenocarcinoma cell lines (Murakami et al., 2002). Murakami et al. (2003a) demonstrated that zerumbone has a marked suppressive effect on DSS-induced colitis in mice. It has recently been found to suppress a tumor promoter, 12-O-tetradecanoylphorbol-13-acetate (TPA)-induced Epstein Barr virus activation in a potent manner (Murakami et al., 2002).

Zerumbone lowered the number of AgNORs in colonic crypt cell nuclei. These findings might suggest possible chemo-preventive ability of Z II, through suppression of COX-2 expression, cell proliferating activity of the colonic mucosa and induction of phase II detoxification enzymes in the development of carcinogen-induced ACF (Tanaka et al., 2001).

Cervical cancer continues to have a devastating impact on women worldwide although a wide population screening in most Western countries has lead to a remarkable reduction in the incidence and mortality of this disease (Segnan, 1994). Approximately 400,000 women developed cervical cancer with 250,000 of the cancer-related deaths reported each year (Parkin et al., 1999).

On the other hand, ovarian cancer is the number one gynaecologic killer in the Western world and is the fourth most common malignancy in women (Greenlee et al, 2000). Approximately 1 in 75 women in the developed world will be affected by ovarian cancer with at least two thirds of women presenting late with the disease because of non-specificity symptoms and often a delay in the diagnosis (Quinn, 2003).

Cisplatin has consistently been proven to be the most effective single cytotoxic agent for the treatment of advanced or recurrent cervical cancer. However, the response rate is between 23% and 50%, and therefore the need of new active drugs is necessary to improve the outcome for patients with cervical cancer (Savarese and Cognetti, 2003). In addition, platinum-based chemotherapy is the most effective treatment in epithelial ovarian carcinoma (Piccart *et al.* 2000).

The prior art remains deficient in the lack of naturally-occurring phyto-compounds capable of inhibiting human carcinoma in cervix and ovaries. Moreover, the prior art is deficient in the lack of an effective method to treat patho-physiological states and having various adverse drug reactions.

In view of the above and the increasing number of patients with various illnesses, everyone is hoping for better medication with fewer side effects. Therefore, it would be advantageous to provide a natural compound having an effect of inducing apoptosis of cancer cells and inhibiting cancer metastasis, as well as being more effective in inhibiting the growth of cancer cells. Also, the compound has not toxicity and is thus capable of being used as a functional food for preventing and treating cancer.

It is an object of the present invention to provide an alternative medicament for the treatment of cervical and ovarian carcinoma. The present invention provides a natural based compound having potent anti-cancer properties which overcome the disadvantages of the methods of therapeutic treatment for cancer in the state of the art.

### SUMMARY OF THE INVENTION

According to a first aspect of the present invention, there is provided the use of a compound having the following formula for the production of a pharmaceutical preparation to prevent and treat cancer of the ovary or cervix:

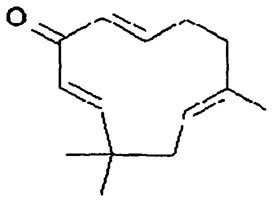

Accordingly, the compound of the present invention provides the desired therapeutic effect to inhibit the proliferation of carcinoma cells of the cervix and ovaries by way of apoptosis mechanism.

A method for extracting and purifying the above-mentioned compound includes the preparation a non-polar extract of Zingiber genus plant, fractionating the non-polar extract through a silica gel column and eluted sequentially with an organic solvent and a mixture of organic solvents to obtain a fraction of a non-polar extract of Zingiber genus plant and purifying the compound by way of recrystallization, partition and chromatographic methods.

The plant is selected from the group consisting of *Zingiber zerumbet* and *Zingiber aromaticum.*

The organic solvents can be selected from the group consisting of hexane and/or ethyl acetate.

According to yet another aspect of the present invention there is provided a pharmaceutical preparation containing the above-mentioned compound as an active ingredient in an amount effective to having potent anti-cancer properties and thus useful in the prevention and treatment of conditions involving cervical and ovarian carcinoma.

The compound can act as a chemotherapy agent inducing a selective cytotoxicity towards cancer cells by way of an apoptosis mechanism.

The pharmaceutical preparation of the present invention is provided in an acceptable carrier and can be administered by any method known to one of ordinary skill in the art such as powder, granule, tablet, capsule, aqueous medicine or injection.

The foregoing compound of a fraction derived from a non-polar organic extract of Zingiber genus plant can be formulated into various pharmaceutical formulations for clinical use such as capsules including soft gel capsules, tablets, galenicals, powder, granules, aqueous medicine, injection and the like by standard methods, in which the compound is present and administered as active component at an effective therapeutic amount based on its efficacy and toxicity, alone or in combination with other chemicals through various routes of administration such as oral, sublingual, intravenous, intramuscular and the like. The effective amount is sufficient to increase and/or induce cytotoxicity towards cancer cells, particularly cervical and ovarian carcinoma, by way of an apoptosis mechanism.

The effective amount to increase and/or induce cytotoxicity will depend on the severity of the condition being treated; individual patient parameters including age, physical condition, size and weight; concurrent treatment and drug interaction; frequency of treatment; and the mode of administration.

### BRIEF DESCRIPTION OF THE DRAWINGS

Figure 1 illustrates a high-pressure liquid chromatography (HPLC) analysis of zerumbone extract according to the present invention;
Figure 2 illustrates a Liquid Column Mass Spectrometry (LCMS) analysis of zerumbone extract according to the present invention;
Figure 3 illustrates the cytotoxicity effects of zerumbone on cervical cancer cells (HeLa);
Figure 4 illustrates the cytotoxicity effects of cisplatin on cervical cancer cells (HeLa);
Figure 5 illustrates the morphological changes of ovarian carcinoma cells (Caov-3) treated with zerumbone and cisplatin at IC₅₀ value after 72 hours (magnification 20x10), control (A), zerumbone (B) and cisplatin (C);
Figure 6 illustrates the morphological changes of cervical cancer cells (HeLa) treated with zerumbone and cisplatin at IC₅₀ value after 72 hours (magnification 20x10), control (A), zerumbone (B) and cisplatin (C);
Figure 7 illustrates the confocal morphological study of human ovarian carcinoma cells (Caov-3) untreated (Control) after (A) 24 hours; (B) 72 hours;
Figure 8 illustrates the morphological study of human ovarian carcinoma cells (Caov-3) with 18.5 mM zerumbone after (A) 24 hours; (B) 48 hours;
Figure 9 illustrates the morphological study of human ovarian carcinoma cells (Caov-3) with 18.5 mM zerumbone (A) 48 hours; (B) 72 hours;
Figure 10 illustrates the morphological changes of cervical cancer cells (HeLa cells) following zerumbone and cisplatin treatment at 48 hours as viewed under laser scanning confocal microscopy after staining with AO/PI. Magnification is 100x10, control (A), zerumbone (B), and cisplatin (C). Arrow B: blebbing of the cell membrane, N: nuclear condensation with margination and nuclear damage;
Figure 11 illustrates the cell cycle progression of cervical cancer cells (HeLa cells) following treatment with Zerumbone at concentrations ranging from 0 to 45 µm;
Figure 12 illustrates zerumbone and cisplatin induced apoptosis in ovarian carcinoma cells (Caov-3 cells) as treated with IC₅₀ values for 24 and 48 hours, respectively. Apoptotic cells were quantified manually after TUNEL Assay. Data shown are mean ± SD from duplicate samples. A single dagger denotes a P of <0.05 when compared with control; a double dagger denotes a P of <0.01 when compared with control as performed by One-Way ANOVA;
Figure 13 illustrates zerumbone and cisplatin induced apoptosis in cervical cancer cells (HeLa cells) as treated with IC₅₀ values for 24 and 48 hours, respectively. Apoptosis was quantified manually after TUNEL Assay. Data shown are mean ± SD from duplicate samples. A double dagger denotes a P of <0.01 when compared with control as performed by One-Way ANOVA;
Figure 14 is a micrograph showing the cervical epithelial cells with low nuclear to cytoplasmic ratio, with no apparent angiogenesis in normal mice;
Figure 15 is a micrograph showing the treatment of induced cervical cancer in female balb/c mice with 10mg/kg cisplatin. Mild dysplasia (CIN 1), with mild nuclear atypia confined to one third of the epithelial layer at magnification 10 x 20;
Figure 16 is a micrograph showing the treatment of induced cervical cancer in female balb/c mice with 16mg/kg zerumbone. Mild dysplasia (CIN I), mild nuclear atypia confined to one third of the epithelial layer (Black arrows) and angiogenesis (Blue arrow) at magnification 10 x 20;
Figure 17 is a micrograph showing the treatment of induced cervical cancer in female balb/c mice with normal saline. Severe dysplasia (CIN III), with severe nuclear atypia confined to the whole layer of the epithelia and severe angiogenesis at magnification 10 x 20; and
Figure 18 is a histogram showing the concentration of serum IL-6 according to treatment groups in mice. The values are significant with P < 0.01 analyzed by One-Way ANOVA using SPSS version 12.

### DETAILED DESCRIPTION OF THE INVENTION

The plant of *Zingiber zerumbet* is characterized by long narrow leaves arranged oppositely along the stem and grows to about 7 feet tall. This plant species is native to Southeast Asia but has been widely cultivated in tropical and subtropical areas around the world, and has neutralized in some areas. In general, several species of Zingiberceae are used as spices, medicines, flavoring agents and as a source of certain dyes in the Southeast Asian region.

With regard to the processing of the raw plant material, it has been determined that the active components or sesquiterpene zerumbone, are localized to one region of the plant. Portion of the plant from which the active components are extracted is the rhizome, which is characterized as a thick stem and grows along or under the ground and has roots and shoots growing from it. In practicing the present invention, the rhizome, portion of the plant is removed and then utilized individually or collectively.

Various features and aspects of the present invention are illustrated further in the examples that follow. While these examples are presented to show one skilled in the art how to operate within the scope of this invention, they are not to serve as a limitation on the scope of the invention where such scope is only defined in the claims.

### Example 1

### Preparation of Code Extract

5 kg of *zingiber zerumbet* plant was cut into small slices, i.e. 1 to 2 mm, and dried in an oven at 37°C for 3 weeks. The dried *zingiber zerumbet* slices were grinded and 432 g of the grinded *zingiber zerumbet* sample was soaked in 100% methanol for 3 days. The sample was then filtered using filter paper. Methanol with several compounds including zerumbone was collected in a conical flask and the remaining sample was soaked again in 100% methanol for the second time and the methanol plus extract were subjected to evaporation at 40°C using a rotor evaporator machine. This step was repeated twice and the final filtered sample and extract were mixed with the previous sample (methanol + extract) and was evaporated to obtain a crude extract in an amount of 40.3 g, which appears as dark brown gum.

### Example 2

### Preparation of Non-Polar Extract

The crude extract was mixed with methanol and distilled water, and then poured into a separation funnel. 100% hexane was added into the separation funnel and the funnel was shaked vigorously to make sure the solutions were mixed well and left for 5 minutes for the solution to be separated into 2 layers, whereby the upper layer is an aqueous layer containing polar compound, while the lower layer is a non-polar layer containing non-polar compound, i.e. zerumbone. Next, the aqueous and nonaqueous layers were collected in separate conical flasks and subjected to separation. The aqueous layer was poured gain into the separation funnel and 100% hexane was added and the separation process was repeated again for 3 times. Then, the entire non-polar layer from the separation process was added together and poured into a round bottom flask and undergone evaporation by a rotor evaporator to produce a non-polar extract containing zerumbone in an amount of 20.6 grams.

### Example 3

### Preparation of a Fraction of Non-Polar Extract

The fractionation process was carried out using column chromatography procedures. It comprises two main packing, which were dry packing and column packing. In the dry packing, the rotor-evaporated extract (20.6 g) was added with silica gel and then rotor-evaporated again until it forms a dried powder (20.0g). In column packing, silica gel was mixed with hexane and poured into the column for chromatography.

Next, the powdered sample in an amount of 20.0 grams was poured into a silica gel column (3.5cm X 30.0cm) which acts as an adsorbent. 100% hexane was added to the silica gel column, which was followed by the addition of a mixture of hexane and ethyl acetate as an eluant in a ratio of 9:1 and finally 8:2. The ratio of 8:2 was maintained and repeated until a non-polar fraction containing zerumbone was isolated from the column. The isolated fractions from the column chromatography were collected into small (10ml) vials.

### Example 4

### Isolation of Purified Zerumbone

The fractions collected in the vials were allowed to dry to obtain crystals. The crystals obtained were subjected to recrystalization to obtain purified zerumbone crystals. These crystals were then washed with hexane to remove unwanted compounds and leaving the pure zerumbone crystals in the vials. Re-crystallization was repeated about 3 times. After re-crystallization, pure zerumbone crystals were dried and collected in clean vials. The amount of pure zerumbone crystals isolated from 5kg fresh zingiber zerumbet plant was about 3.1g.

The constituents collected in the fractions were determined using thin layer chromatography (TLC). The TLC of the fractions was compared with the TLC of pure zerumbone to locate in which fractions the zerumbone was collected. The yield of zerumbone obtained is shown in the following table.

High performance liquid chromatography (HPLC) was performed using an auto sampler and quaternary solvent pump. The separation was done using an analytical reverse phase column (C-18 column). A mixture of 40% water + 60% Acetone nitrile was used as a mobile phase to separate the compound at a flow rate of 0.2ml/min.

Referring to Figure 1, HPLC analysis of the sample solution showed only one single high peak with a retention time of 16 minutes. This single peak ensures purity of the compound isolated, which exemplified further that the compound extracted and isolated from crude plant extract consisted mainly as one single compound. Though a single peak was obtained following HPLC analysis, further analysis using liquid chromatography mass spectrometer (LCMS) was needed in order to identify the molecular weight of the compound isolated.

LCMS was performed using an auto sampler and a quaternary solvent pump. The separation was done using an analytical reverse phase column, C-18. A mixture comprising of 40% (v/v) 10mM ammonium acetate and 60% (v/v) acetone nitrile was used as mobile phase to separate the compound at a flow rate of 0.2 ml/min. A liquid chromatography system was connected to an ion trap mass spectrometer, consisting of an Atmospheric Pressure Chemical Ionization (ACPI) interfaced in positive ion mode, linking to a personal computer that has a data acquisition / processing software system.

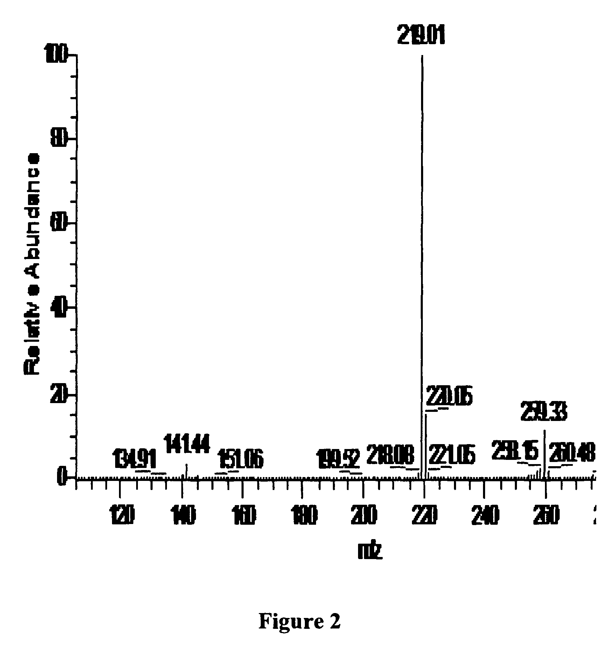

The mass spectra of zerumbone were studied in positive ionization mode in a LCMS analysis graph, as illustrated in Figure 2. The most intense ions in the full mass spectrum are the protonated ([M + H]⁺) molecules. Referring to Figure 2, the LCMS analysis showed that the compound had generated a high fragment with molecular ions at m/z 219 [M + 1]+ under positive scan modes. The molecular weight of this fragment can be derived from the positive ions (positive ions: 219.01 - 1 = 218.01). Base on this calculation, the molecular weight of the fragment is 218, which is the molecular weight of the compound, zerumbone. This fragment is the most abundant in Mass Spectrometry graph. The compound also generated a fragment ion under positive product ion scan mode (MS/MS) at m/z 259, which can be assigned as the product ion due to a loss of a molecule from the precursor ion. In the following analysis conducted, only product ions with relative abundance greater than 25% in the spectra were considered. Since the fragment at m/z 259 has relative abundance less than 25%, it is not considered. This finding duly confirms that the compound isolated is indeed, pure zerumbone crystals.

**Table 1**

| | | | |
|---|---|---|---|
| Yield of Zerumbone | | | |

| Weight of dried plant material, g | Weight of hexane crude extract, g | Weight of pure zerumbone, g | Percentage yield, % |
|---|---|---|---|
| 432.0g | 40.3g | 3.1g | 0.06% |

### Example 5

### Identification of Zerumbone Compound

Spectroscopy analysis of ¹H-NMR and ¹³C-NMR was carried out on the zerumbone compound. To the applicant's knowledge, there has been no previous report published on its spectral data.

The ¹H-NMR spectral data in Table 2 shows two ubiquitous peaks of dublet dublet and dublet that revealed the meta coupled signals at δ 5.968 and 6.106, one of which displayed a long-range coupling of 0.97 Hz with a signal at δ 2.43 detected as a triplet. Further, there were signals due to a methoxy group at δ 3.22, long alkyl side chain at δ 1.086-1.573 (m, 16H, H-3'-H10') and δ 1.770 (s, 1H, H-2').

**Table 2 ¹H-NMR data of Zerumbone**

| H Position | Multiplicity | Chemical Shift (δ) |
|---|---|---|
| H1, 4 & 5 | Multiplet | 2.243-2.544 |
| H-6 | Broad duplet | 6.083-6.106 |
| H-14 | Singlet | 1.086 |
| H-15 | Singlet | 1.238 |
| H-12 | Singlet | 1.573 |
| H-13 | Singlet | 1.770 |

IR: vmax cm-1: 1654.8 (Conjugated C=O), 1386.7 and 1341.7 (gem-dimethyl). 1H NMR (CDCI3): 1.04 (3H, s, H-14), 1.17 (3H, s, H-15), 1.51 (3H, s, H-12), 1.77 (3H, s, H-13), 1.87 (1H, d, *J*=12.8 Hz, H-11), 2.19-2.44 (5H, m, H-1, H-4 and H-5), 5.22 (1H, bd, *J*=14.8 Hz), 5.83 (1H, d, *J*=16.4 Hz), 5.95 (1H, d, *J*=16.4 Hz), 5.99 (1H, bd, *J*=12.4 Hz, H-6).

The carbon positions of zerumbone are shown in Table 3. The ¹³C-NMR spectrum shows signals indicative of a methoxylated 1,4-benzoquinone at δ 39.210 (s, C-4), 42.142 (s, C-1), 124.905 (s, C-2), 149.271 (s, C-6), 24.261 (s, C-5) and 136.457 (s, C-3).

**Table 3**

| | | |
|---|---|---|
| ¹³C-NMR Data of Zerumbone | | |

| C Position | Type of Carbon | Chemical Shift (ppm) |
|---|---|---|
| C1 | RCH₂ | 42.142 |
| C2 | RCH | 124.905 |
| C3 | RC | 136.457 |
| C4 | RCH₂ | 39.210 |
| C5 | RCH₂ | 24.261 |
| C6 | RCH | 149.971 |
| C7 | RC | 137.747 |
| C8 | RC=O | 205.538 |
| C9 | RCH | 126.932 |
| C10 | RCH | 161.942 |
| C11 | RC | 37.683 |
| C12 | RCH₃ | 14.228 |
| C13 | RCH₃ | 10.724 |
| C14 | RCH₃ | 23.360 |
| C15 | RCH₃ | 28.659 |

13C NMR (CDCI3): 11.76 (C13), 15.18 (C-12), 24.16 (C-14), 24.36 (C-5), 29.39 (C-15), 37.83 (C-11), 39.40 (C-4), 42.36 (C-1), 124.94 (C-2), 127.11 (C-9), 136.25 (C-3), 137.9 (C-7), 148.84 (C-6), 160.75 (C-10), 204.37 (C-8).

### Chemical Structure of Zerumbone Compound

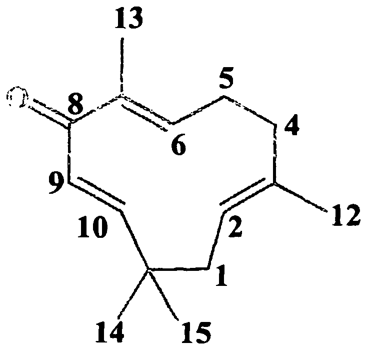

### Example 6

### Evaluation of the Effect of Zerumbone on Cervical Cancer Cells (HeLa) and Ovarian Cancer Cell (Caov-3)

### (i) Cell Culture

The cancer cell lines used in this study were cervical cancer cell lines, HeLa (ATCC Number: CCL-2) and ovarian cancer, Caov-3 (ATCC Number. HTB-75) of human origin. HeLa and Caov-3 cell lines were maintained in RPMI 1640 and DMEM high glucose respectively, supplemented with 10% FCS, 1% penicillin and 1% streptomycin. Cells were allowed to proliferate as monolayer cultures in 25 cm² and 75 cm² tissues culture flasks, in a humidified atmosphere containing 5% CO₂ at 37°C.

### (ii) Cell Plating

A haemacytometer was used to determine the concentration of cells in the flask. Confluent cells were trypsinised in order to detach cells from surface of the flask. 10 mL of fresh medium was added into the flask and the cells were suspended thoroughly. 10µl of medium from the flask was then transferred into a haemacytometer with cover slip. The counting was done under an inverted microscope and facilitated by manual counter usage. The cell density (for an example 1x10⁵ cells/mL) was calculated. Using a multi-channel pipette, 100µl of cell culture solution was transferred into each well of a 96-well microtiter plate. For a 6-well microtiter plate, 2 ml of the cell culture solution was pipetted instead. The plate was incubated overnight at 37°C in 5% CO₂.

### (iii) Treatment of Cells

After an overnight incubation of both the human cancer cells, the cells were treated with zerumbone. The concentrations of zerumbone in each experiment varied according to the optimization of the experiment conducted. The controls or untreated cells received only medium without any treatment of either compounds. Treated cells were then incubated at 37°C in 5% CO₂.

### (iv) Micro Tetrazolium Cytotoxicity Assay (MTT Assay)

This assay is based on the metabolic reduction of soluble MTT by mitochondrial enzyme activity of viable cancer cells, into an insoluble coloured formazan product, which can be measured spectrophotometrically after dissolution in dimethyl sulfoxide (DMSO) (Carmichale *et al.,* 1987). The MTT assay was conducted according to the methods of Kumi-Diaka *et al*. (1998) with minor modifications. The concentration of MTT solution used was 5 mg/mL. The MTT solution was prepared by dissolving 250 mg of MTT powder in 50 mL of phosphate buffer saline (PBS). A 0.2 micron syringe filter was used to filter the dissolved MTT. The filtered MTT solution was transferred into a sterile 50 mL centrifuge tube and covered with aluminum foil. This solution was stored in 4°C and is ready to be used. The MTT assay was carried out as follows:
96-well mictotiter plates containing cell culture solution were removed from the incubator after 72 hours incubation. 20 µl of 5 mg/mL MTT solution was added into each well including control wells. The plates were then wrapped with aluminium foil and incubated further for 4 hours at 37°C in 5% CO₂. After incubation, excess MTT reagent was aspirated and 100 µl of DMSO was added to dissolve the remaining purple colour formazan crystals. The plates were gently mixed on a plate shaker for approximately 4 minutes. The absorbance of the formazan solution was determined at 450 nm using a microplate reader.

Micro-Tetrazolium (MTT) cytotoxicity assay was performed on human cervical cancer cells, HeLa after treatment with compounds, zerumbone and cisplatin. An IC₅₀ value ≤4 ug/ml (≤18µM) is considered being very significant whilst, IC₅₀ value in between 4 and 30 ug/ml (18 - 137.6 µM) is considered to be significant, based on the National Cancer Institute Chemotherapeutic Standard, United States (Geran et al., 1972).

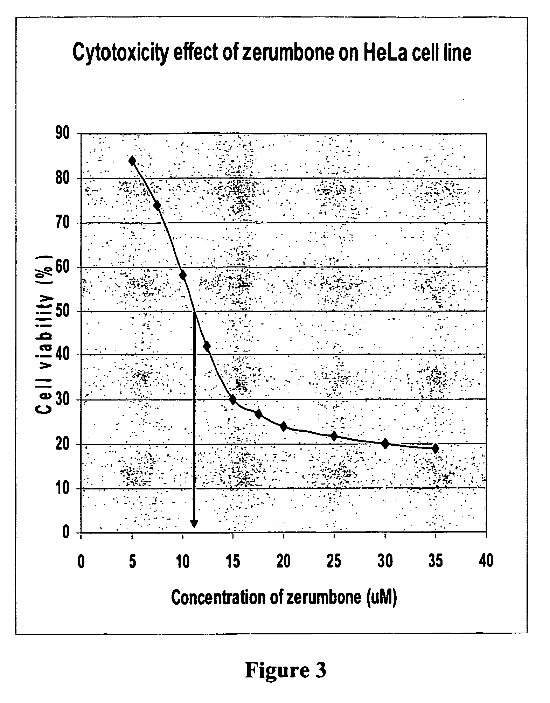

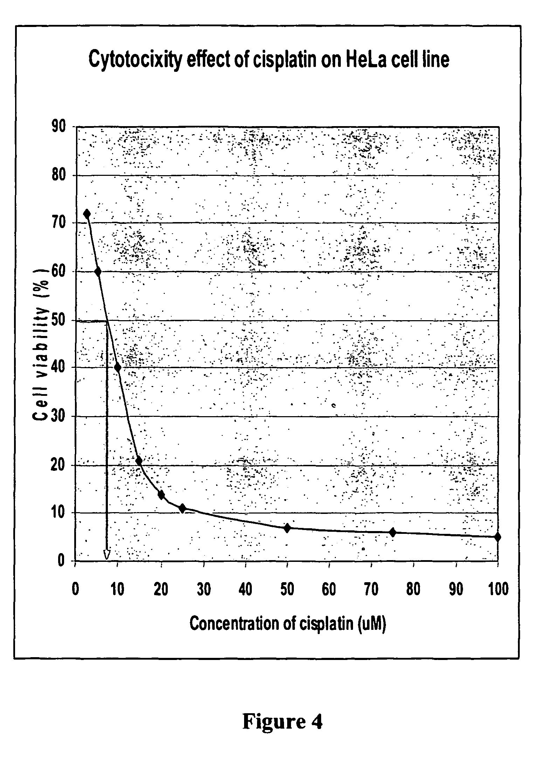

Figures 3 and 4 illustrate the cytotoxicity effects of zerumbone and cisplatin respectively on HeLa cancer cells. Figure 3 shows the effects of zerumbone to inhibit growth proliferation of human cancer cells, HeLa after 72-hours post-treatment of the compound at concentrations, 5µM to 35µM. Referring to Figure 3, the IC₅₀ value (± S.E.M) of zerumbone was determined to be 11.3 µM (2.5 ug/ml).

Figure 4 illustrates the effects of cisplatin to inhibit growth proliferation of human cancer cells, HeLa after 72-hours of post-treatment with the compound at concentrations, 2.5µM - 100µM. Referring to Figure 4, the IC₅₀ value (± S.E.M) (P < 0.01) of cisplatin was determined at 7.5 µM ± 0.3 (1.6 ug/ml).

The results showed that cisplatin exerted better cytotoxic effects on HeLa cancer cells at lower concentration (7.5 µM) as compared to zerumbone, which needed higher concentration (11.3 µM) to induce similar effects. There is however, a significant difference in the IC₅₀ value of both zerumbone and cisplatin on HeLa cancer cells with (P < 0.01) as analyzed using Independent sample t-Test. Even though cisplatin have lower IC₅₀ value compared to zerumbone, nonetheless, both IC₅₀ values of zerumbone and cisplatin fall within the very significant range (≤4 ug/ml) of cytotoxic effects, as established by the National Cancer Institute Standard, United States, 1972.

### (v) Morphological Studies Using Normal Inverted Microscope

Treated cancer cells of HeLa and Caov-3 in 96-well plates were observed under normal inverted microscope following 24, 48 and 72 hours of incubation. Apoptotic features of treated cells were examined and compared concurrently to untreated cells. The cells were observed under inverted microscope at magnification 20x10.

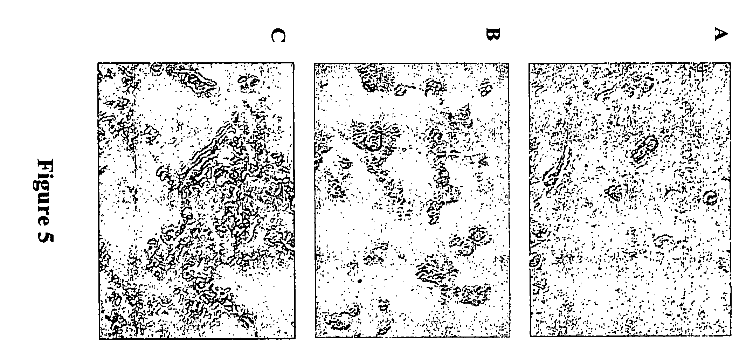

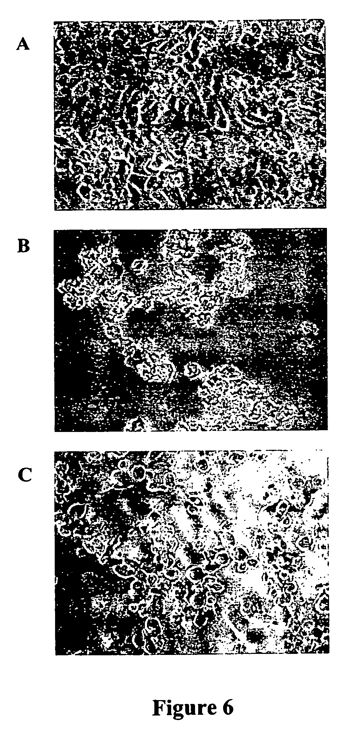

Significant morphological changes on the HeLa cancer cells and Caov-3 cancer cells were observed during treatment with zerumbone and cisplatin, after 24-hours, 48-hours and 72-hours post-addition. Morphological changes on Caov-3 cancer cells are illustrated in Figure 5, whereby the cells were treated with control (A), zerumbone (B) and cisplatin (C). On the other hand, morphological changes on HeLa cancer cells are illustrated in Figure 6, whereby the cells were treated with control (A), zerumbone (B) and cisplatin (C).

Prominent growth retardation was identified in the Caov-3 cancer cells (see Figure 5) and HeLa cancer cells (see Figure 6) after treatment with zerumbone and cisplatin, as compared to untreated control cells, observed to have rapid cell growth. At 24-hours post-treatment, few rounded cancer cells (cell shrinkage) were observed after zerumbone treatment, whilst the cancer cells treated with cisplatin showed more rounded cancer cells with budding cell membrane. An increased number of rounded cancer cells were observed at 48-hours post-treatment with zerumbone. At similar time interval however, the HeLa cancer cells and Caov-3 cancer cells treated with cisplatin showed even more abundant rounded cells as compared relatively to zerumbone. A progressive nuclear shrinkage with increased rounded cancer cells was noticeable to the HeLa cancer cells and Caov-3 cancer cells treated with zerumbone at 72-hours post-treatment, whereas the 72-hours post-treatment with cisplatin resulted in higher increased of rounded cells.

### (vi) Confocal Microscopic Studies Using Acridine Orange and Propidium Iodide (AO/PI) Stain

Each cancer cell lines were plated in 6-well tissue culture plates and treated either with zerumbone or cisplatin. The plates were removed from the incubator after 24 and 48 hours of incubation period. The medium containing cells was transferred into labeled Eppendorf tubes (of each treated samples) placed in ice. The remaining cells in the wells were trypsinised until all cells were detached from the surfaces of each well. The medium containing cells in the Eppendorf tube was then transferred into the well of the tissue culture plate (of similar sample) to neutralise the trypsin. The medium in the wells was later transferred back into the Eppendorf tube (of similar sample) and centrifuged at 3000 rpm for 5 minutes, 4°C. Supernatant was discarded and 1 mL of 70% of cold alcohol was added. The pellet was re-suspended and placed in ice for 15 minutes for fixation. The samples were then centrifuged again at 3000 rpm for 5 minutes, 4°C. The supernatants containing the alcohol were discarded. The pellet was then re-suspended in 1 mL of phosphate buffer saline (PBS) and placed in ice for 10 minutes. The samples were centrifuged again, re-suspended in 1 mL of PBS and placed in ice for 10 minutes. This procedure was repeated again before discarding the supernatant

The pellet containing cells was re-suspended with 15 µl of 10 µg/mL AO/PI mixture. Approximately 10 µl of the cell suspension was placed on a glass slide, and anti-fading agents were added onto the cells. The slides were covered with cover slips and nail polish was used to seal the ends of the cover slip. The slides were viewed at magnification 100x10 under oil immersion of laser scanning confocal microscopy. The nuclei of non-viable (dead) cells whose plasma membranes were leaky were labeled with propidium iodide. Propidium iodide enters only cells that are non-intact having disrupted membranes.

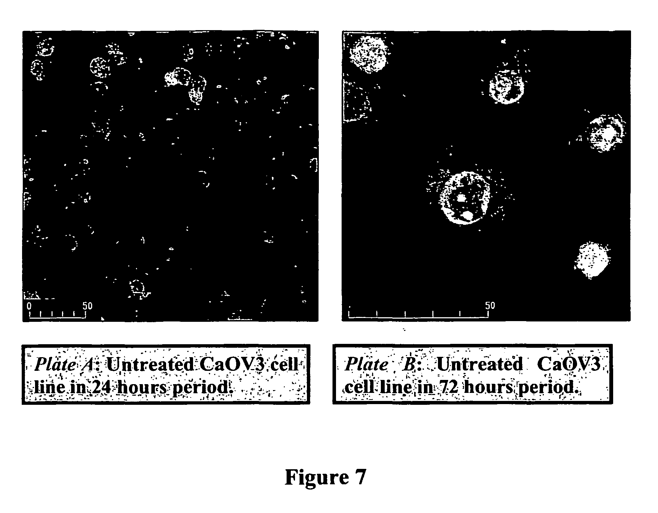

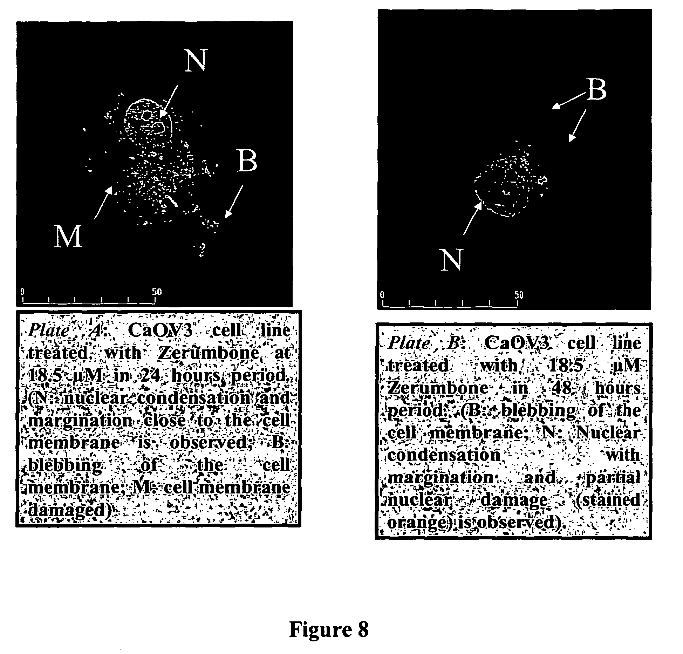

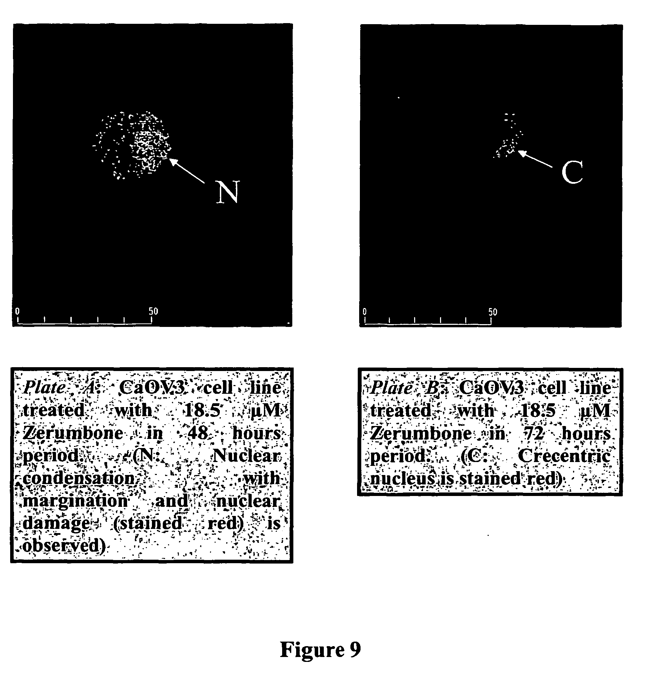

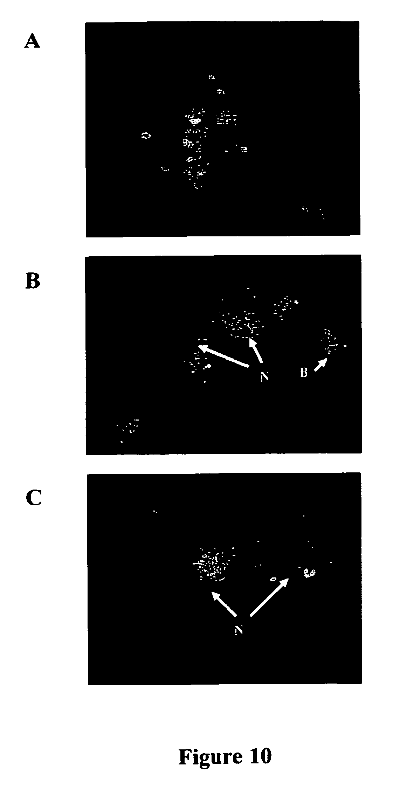

Figures 7, 8 and 9 show the morphological changes of Caov-3 cancer cells and Figure 10 shows the morphological changes of HeLa cancer cells following zerumbone and cisplatin treatment at 48 hours as viewed under laser scanning confocal microscopy after staining with AO/PI. Magnification at 100x10, control (A), zerumbone (B), and cisplatin (C). Zerumbone (B) and cisplatin (C) showed blebbing of the cell membrane, nuclear condensation with margination and nuclear damage.

### (vii) Cell Cycle Studies Using Flow Cytometry

Flow cytometry analyses of HeLa cells were conducted after 24 hours incubation at concentrations of 15, 25, 35 and 45 µM zerumbone and 15, 25, and 35 µM cisplatin. Cells were plated in 25 cm² tissue culture flasks at concentrations of 0.5 x 10⁵ cells/mL. After an overnight incubation, the cells were cultured in the presence of zerumbone at concentrations as indicated above for 24 hours. Cells were harvested using trypsin-EDTA before centrifuging at 3000 rpm for 5 minutes. The cells pellet was then re-suspended in 2 mL of phosphate buffer saline (PBS). The re-suspended cells were centrifuged at 3000 rpm for 5 minutes and the resulting cells pellet was re-suspended again in 1 mL of cold PBS. The cells were fixed by the addition of 4 mL of cold absolute ethanol (kept in -20°C prior to use). The fixed cells were stored in - 20°C overnight before resuming the staining procedure.

### Observation on HeLa cells

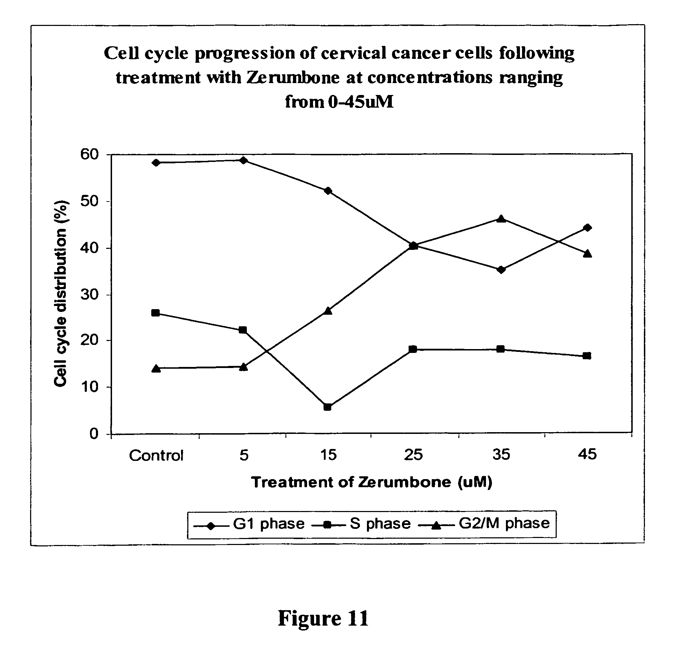

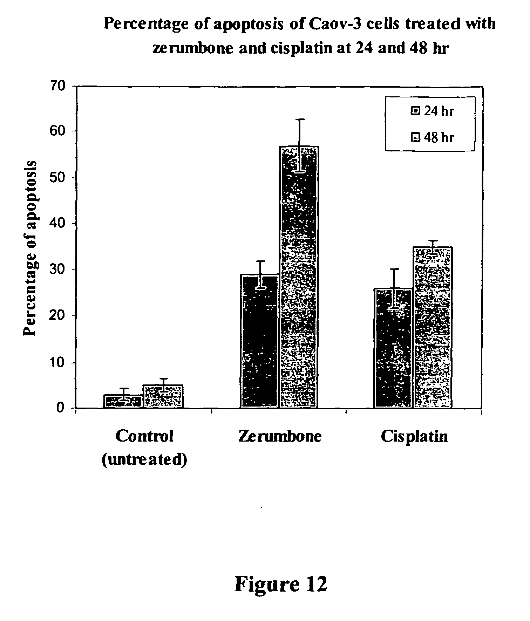

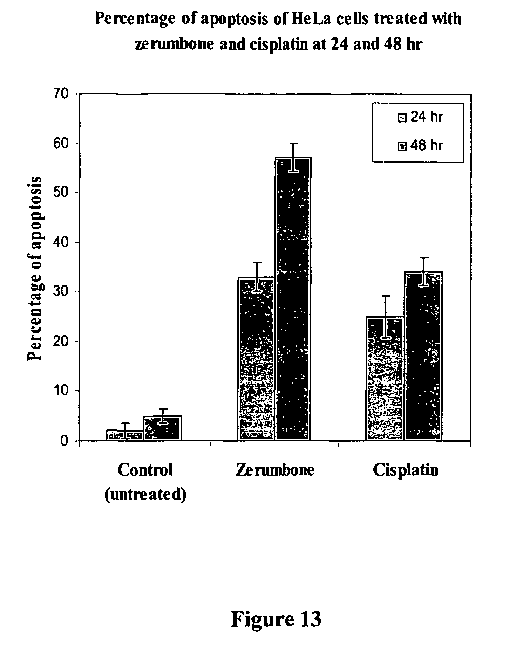

As referred to Figures 11, 12 and 13, cell cycle analysis showed an increase in the number of HeLa cancer cells and Coa3 cancer cells in the G₂/M phase and a decrease in the G₁ phase and S phase. Data shown are mean ± SD from two different experiments. A single asterisk denotes a *P* of <0.05 when compared with control; a double asterisks denotes a *P* of <0.01 when compared with control; a triple asterisks denotes a *P* of <0.001 when compared with control as performed by one-way ANOVA.

### (viii) Induction of Cervical Cancer in Mice

1 male mouse was cohabitate with 3 female mice for 48 hours prior to experiment to stimulate the ovulation of the female mice. After 48 hours, 1 male mouse was cohabitate with 1 female mouse. The next morning, the female mice were checked for a vaginal plug. The detection of vaginal plug at the vagina of the female mice were considered as the first day of pregnancy and gestation day (G = 0). Once the female mouse was pregnant the corresponding male mouse was separated from the cage. Only one pregnant female mouse was placed in each cage to reduce the stress among the mice and also to reduce the chances of cannibalization of offspring by the mothers. The pregnant mice were given subcutaneous injection of 67 µg/kg body weight diethylstilbestrol (DES) dissolved in sesame oil (refer to appendix for DES preparation) from gestation day (13 to 18). Since the reproductive tract is developing during this time, so giving the DES injection too early may cause stillbirth. The mice gave birth approximately at gestation day (19 to 22). The offspring were weaned at 22 days of age. 1 female offspring was subjected for the confirmation of cervical dysplasia at 41 days of age. The other offspring were subjected for the treatment with cisplatin, zerumbone and normal saline.

All female offspring exposed to DES in uteri were pooled together and were divided into 4 groups where each group consists of 3 mice. The 5^{th} group consists of 3 mice which was non-exposed to DES. The mice were given treatment starting from 46 days of age to 52 days of age. The treatment was given every alternate day (day 46, 48, 50, 52) which consist of 4 dosages. The treatment was given through intra peritoneal injection. The group 1 mice were treated with 0.9% of Normal Saline and this group acts as a positive control group. The group 2 mice were given 8mg/kg of Zerumbone while the group 3 mice were given 16mg/kg of Zerumbone. 10mg/kg of Cisplatin was given to the mice in group 4. Finally no treatment was given to the mice in group 5 as they act as a negative control (normal mice) for the studies. Following treatment, all the mice were sacrificed at 54 days of age. The blood sample was collected through heart punctured method into a plain tube and was centrifuged at 3000rpm for 15 minutes. The supernatant (serum) was collected into an eppendorf tube and was immediately stored in ice (0°C - temporary storage). Once serum from all the samples was collected, the serum was stored at -20°C. Then the mice were dissected. The reproductive organ of the mice were collected and washed with 0.9% normal saline. The washed organ was soaked in 10% formalin and stored at room temperature.

The mice were sacrificed at 54 days of age and the cervical tissue was collected. The cervical tissue was then fixed in 10% formalin for 4 weeks. The cervical tissue was processed using tissue processor machine (Leica TP 1020-1-1). The processed tissue was then embedded in paraffin wax using embedding machine (Leica EG1160 TP 1020). The embedded tissue blocks were sectioned using microtome (Leica RM 2145). The sections were stained using Hematoxylin and Eosin staining method.

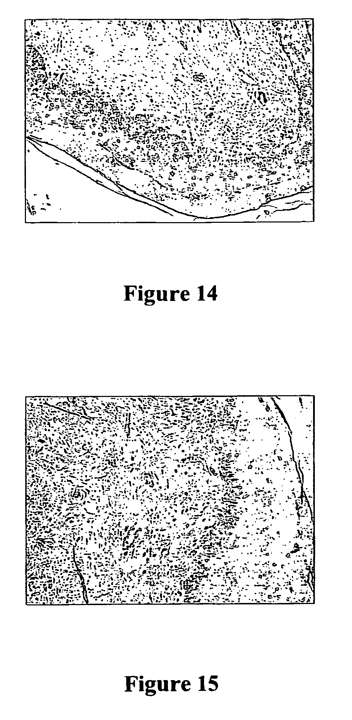

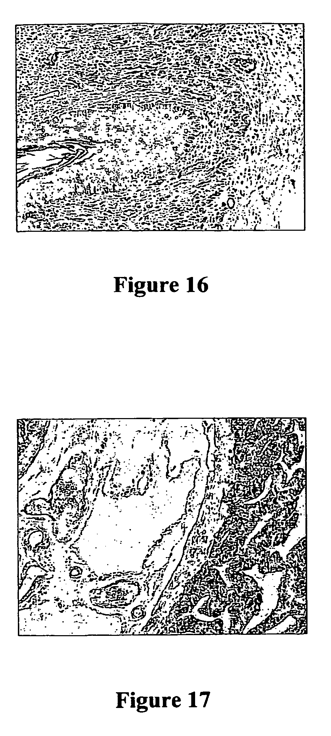

Referring to Figure 14, tissue sections of normal tissues showed normal epithelial arrangement with low nuclear to cytoplasmic ratio but with no presence of angiogenesis. In contrast to the normal saline treated female mice, as shown in Figure 17, cervical tissues of induced cervical cancer in female balb/c mice showed severe dysplastic changes (CIN III). In addition, the cervix tissues also exhibited arrangements of the epithelial cells to be disoriented (confined to whole layer), higher nuclear to cytoplasmic ratio (hyperchromatism) and cytoplasmic swelling and clarity with marked angiogenesis. Treatment of cervical cancer induced in female balb/c mice with 16 mg/kg of zerumbone, as shown in Figure 16, and 10 mg/kg of cisplatin, as shown in Figure 15, showed mild dysplastic changes (CIN I), with mild cytoplasmic clarity, disoriented epithelial cell arrangements and mild angiogenesis to the cervix sectioning.

Both cisplatin and zerumbone have demonstrated being able to inhibit the proliferation of mild dysplasia (CIN I) to progress into becoming more severe dysplasia (CIN III). The compound, zerumbone is able to decrease dysplastic progression of the cervical tissue similarly to the reference drug, cisplatin.

### (ix) Evaluation of Zerumbone as Anti-cancer Agent in Cervical Cancer of Mice

Determination and Quantification of serum Interluekin-6 (IL-6)

Serum blood levels of IL-6, from female mice induced cervical cancer, was determined and quantitated using a commercial Anti-Mouse IL-6 ELISA Immunoassay kit after treatment with compounds, zerumbone, cisplatin and normal saline (positive control). The IL-6 serum levels in normal mice were used as negative control.

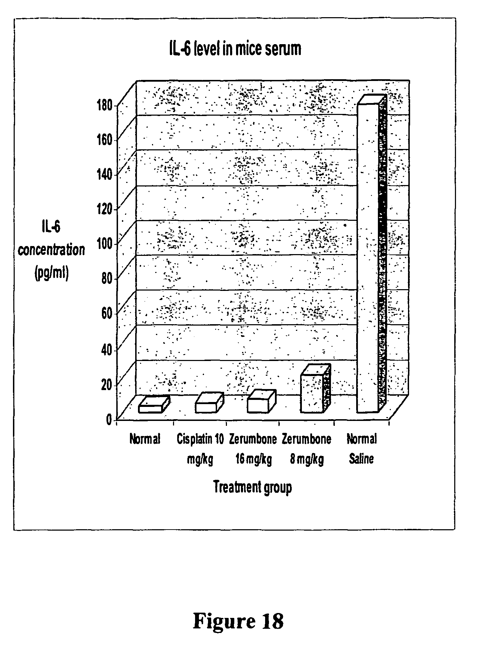

Referring to Figure 18, the serum IL-6 levels of female mice induced cervical cancer treated with zerumbone and cisplatin were lower than mice induced cervical cancer treated with normal saline, with P < 0.01, analyzed by One-Way ANOVA. The results showed that serum IL-6 levels of 16 mg/kg zerumbone and 10 mg/kg cisplatin treated mice were almost near to the serum level of normal mice (negative control). The Post Hoc comparison test (One-Way ANOVA), shows that there is a significant mean difference between serum IL-6 levels of normal saline treated mice with four other treatments groups with P < 0.01. A significant decreased was noticeable in serum IL-6 levels of mice treated with zerumbone and cisplatin (P < 0.01). The result indicates that zerumbone and cisplatin were capable of reducing or inhibiting the secretion of IL-6 almost at the similar rate.

## Claims

1. A compound according to formula (I): 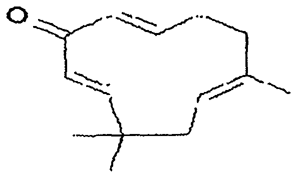 for use in the treatment or prevention of cancer of the ovary or cervix.

2. A compound for use according to claim 1, **characterized in that** the compound provides the desired therapeutic effect by way of inhibiting the proliferation of carcinoma cells of the cervix or ovary.

3. A compound for use according to claim 1, **characterized in that** the compound is obtained from Zingiber zerumbet or Zingiber aromaticum.

4. A compound for use according to claim 1, **characterized in that** the compound is obtained from a plant material including rhizome, stem, root and shoot.

## Patentansprüche

1. Verbindung nach Formel (1): 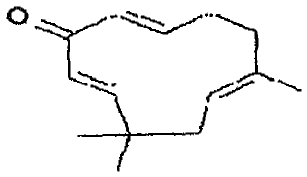 zur Verwendung in der Behandlung oder Prävention von Eierstock- oder Gebärmutterhalskrebs.

2. Verbindung zur Verwendung nach Anspruch 1, **dadurch gekennzeichnet, dass** die Verbindung die gewünschte therapeutische Wirkung durch Hemmen der Vermehrung von Krebszellen des Gebärmutterhalses oder Eierstocks ergibt.

3. Verbindung zur Verwendung nach Anspruch 1, **dadurch gekennzeichnet, dass** die Verbindung aus Zingiber zerumbet oder Zingiber aromaticum gewonnen wird.

4. Verbindung zur Verwendung nach Anspruch 1, **dadurch gekennzeichnet, dass** die Verbindung aus einem Pflanzenmaterial einschließlich Rhizom, Stängel, Wurzel und Trieb gewonnen wird.

## Revendications

1. Composé selon la formule (1) : 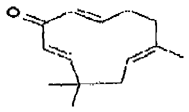 destiné au traitement du cancer de l'ovaire ou du col de l'utérus ou à sa prévention.

2. Composé destiné à une utilisation selon la revendication 1, **caractérisé en ce qu'**il apporte l'effet thérapeutique souhaité en inhibant la prolifération des cellules carcinomateuses du col de l'utérus ou de l'ovaire.

3. Composé destiné à une utilisation selon la revendication 1, **caractérisé en ce qu'**il est obtenu à partir de Zingiber zérumbel ou de Zingiber aromaticum.

4. Composé destiné à une utilisation selon la revendication 1, **caractérisé en ce qu'**il est obtenu à partir d'une matière végétale dont un rhizome, une tige, une racine et une pousse.
